# EUROPEAN PATENT APPLICATION

(11) **EP 1 908 480 A1**
(43) Date of publication of application: **09.04.2008**
(21) Application number: 06747113.6
(22) Date of filing: 02.06.2006
(51) Int. Cl.: A61K 45/00, A61K 31/7105, A61K 48/00, A61P 1/02, A61P 1/04, A61P 1/16, A61P 3/10, A61P 5/14, A61P 7/06, A61P 7/10, A61P 9/00, A61P 11/02, A61P 11/06, A61P 13/12, A61P 17/00, A61P 17/04, A61P 17/06, A61P 19/02, A61P 19/06, A61P 19/08, A61P 21/00

(54) **INTERFERON- ALPHA REGULATOR**

(30) Priority: 03.06.2005 JP 2005163647
(71) Applicant: Riken, Wako-shi, Saitama 351-0198 (JP)
(72) Inventor: KAISHO, Tsuneyasu, c/o RIKEN, Yokohama Institute, Yokohama-shi, Kanagawa 2300045 (JP); HOSHINO, Katsuaki, c/o RIKEN, Yokohama Institute, Yokohama-shi, Kanagawa 2300045 (JP)
(74) Representative: Blum, Erwin
(86) International application number: PCT/JP2006/311072
(87) International publication number: WO 2006/129784

(57) **Abstract**

An object of the present invention is to elucidate the role of IKKα in TLR signaling and provide an agent and a method for controlling interferon-α. The present invention provides an agent for suppressing interferon-α production which comprises an agent for inhibiting IKKα.

## Description

### Technical Field

The present invention relates to an agent for controlling interferon-α and a method for controlling interferon-α. More specifically the present invention relates to an agent or a method for suppressing interferon-α production through the use of an agent for inhibiting IKKα, and an agent or a method for inducing interferon-α production through the use of IKKα or a substance that increases IKKα expression.

### Background Art

Toll-like receptors (TLRs) are expressed on antigen-presenting cells such as dendritic cells (DC) and are involved in recognition of molecular structures derived from various microorganisms. Examples of TLR ligands include lipids, proteins, and nucleic acid components derived from bacteria and viruses. Most TLRs have effects of activating dendritic cells, which are common among TLRs, such as induction of inflammatory cytokines (e.g., IL-6) and expression of co-stimulatory molecules. Each TLR exerts a unique function.

TLR7 and TLR9 can recognize single-chain RNA and unmethylated CpG DNA, respectively. TLRs that recognize these nucleic acids are characterized in that they can induce type I interferon (IFN) required for antiviral immunity, such as IFN-α and IFN-β (Wagner, H, Trends Immunol 25, 381-6 (2004)). TLR7 and TLR9 are associated with MyD88, which is an intracytoplasmic adaptor molecule. MyD88 is required for dendritic cells to produce not only type I interferon, but also inflammatory cytokines in response to TLR ligands. Transcription factor IRF-7 is also important for type I interferon production that is induced by TLR-7/9 (Honda, K et al., Nature, 434, 772-7 (2005)). IRF7 is associated with MyD88 and tumor necrosis factor (TNF) receptor-associated factor 6 (TRAF6), so as to induce an IFN-α gene (Kawai, T. et al., Nat Immunol 5, 1061-8 (2004); and Honda, K. et al., Proc Natl Acad Sci U.S.A. 101, 15416-21 (2004)). Furthermore, it is known that IRAK-1 is contained in this molecule via its association with IRF-7, and it is also known that IRAK-1 is required for IFN-α production mediated by TLR7/9 signaling (Uematsu, S. et al., J Exp Med 201, 915-23 (2005)).

TLR3 and TLR4, which recognize double-stranded RNA and LPS, respectively, can mediate signals for IFN-β gene induction. This induction requires two IKK family factors, TBK-1 and IKK/IKKε (Hemmi, H. et al. J Exp Med 199, 1641-50 (2004); and Perry, A.K., et al., J Exp Med 199, 1651-8 (2004)). These kinases are involved in TLR3/4-induced IFN-β gene expression via their association with Myd88-associated adaptor molecule TRIF (Yamamoto, M. et al. Science 301, 640-3 (2003); and Fitzgerald, K.A. et al. Nat Immunol 4, 491-6 (2003)). However, TLR9-induced IFN-α production is not deteriorated in TBK-1 or IKK 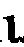 /IKKε-deficient mice, suggesting the involvement of another IFN-α induction pathway (Kawai, T. et al., Nat Immunol 5, 1061-8 (2004)).

IKKα and IKKβ were the first factors which were identified among the members of the IKK family (Hayden, M. S. et al., Genes Dev 18, 2195-224 (2004); and Bonizzi, G. et al., Trends Immunol 25, 280-8 (2004)). IKKβ is essential for the regular pathway of NF-κB activation induced not only by TLR, but also by cytokines. IKKβ is critical for inflammatory cytokine production. IKKα is not essential for this pathway, but it is essential for the non-canocical pathway of downstream NF-κB activation mediated by LT-α, B cell activating factor (BAFF), or CD40. IKKα is essential not only for keratinocyte differentiation, but also for B cell maturation and formation of Peyer's patches. However, the role of IKKα in TLR signaling still remains unknown.

### Disclosure of the Invention

### Objects to Be Achieved by the Invention

An object to be achieved by the present invention is to elucidate the role of IKKα in TLR signaling and provide an agent and a method for controlling interferon-α.

### Means for Attaining the Object

The present inventors have analyzed IKKα-deficient mice and examined how IKKα is involved in the effect induced by TLRs. As a result, the present inventors have discovered that IKKα is required for induction of IFN-α production from dendritic cells via TLR7- and 9-stimulation. Thus they have completed the present invention.

Specifically, according to the present invention, there is provided an agent for suppressing interferon-α production which comprises an agent for inhibiting IKKα.

Preferably, the agent for inhibiting IKKα is a substance that inhibits IKKα expression or an IKKα mutant lacking kinase activity.

According to another aspect of the present invention, there is provided a method for suppressing interferon-α production from cells, which comprises inhibiting intracellular IKKα activity.

According to another aspect of the present invention, there is provided an agent for inducing interferon-α production which comprises IKKα or a substance that increases IKKα expression.

According to still another aspect of the present invention, there is provided a method for inducing interferon-α production from cells, which comprises administering IKKα or a substance that increases IKKα expression to cells.

### Best Mode for Carrying Out the Invention

The embodiments of the present invention will be described below.

The present invention relates to an agent for suppressing interferon-α production which comprises an agent for inhibiting IKKα, and an agent for inducing interferon-α production containing IKKα or a substance that increases IKKα expression. In the description, such an agent for suppressing interferon-α production and agent for inducing interferon-α production may be named generically an agent for controlling interferon-α.

IKKα is a type of kinase and the nucleotide sequence of the gene has already been reported (Accession No. NM_007700; Connelly MA, Marcu KB. CHUK, a new member of the helix-loop-helix and leucine zipper families of interacting proteins, contains a serine-threonine kinase catalytic domain. Cell Mol Biol Res. 1995; 41: 537-49).

Examples of an agent for inhibiting IKKα to be used in the present invention include a substance that inhibits IKKα expression, an IKKα mutant lacking kinase activity, and a substance that acts on IKKα to inhibit the activity and the functions of IKKα. In the description, the term "inhibit" means "suppress" or "reduce".

Examples of such substance that inhibits IKKα expression include substances and the like with the use of RNAi, an antisense method, or a ribozyme method. Examples of the same are not particularly limited, but siRNAs with the use of RNAi are preferred. A specific example of an IKKα mutant lacking kinase activity is a mutant prepared via substitution of lysine residue 44 with an alanine residue. Furthermore, examples of such substance that acts on IKKα so as to inhibit the activity and the functions of IKKα include low-molecular-weight compounds and antibodies.

As an antibody, for example, an antibody prepared with the use of a peptide having full-length or partial sequence of IKKα as an immunogen can be used. As full-length IKKα, recombinant IKKα or the like can be used, for example. Such an antibody can be prepared according to a conventional technique. A monoclonal antibody is preferred herein. An example of such peptide is a peptide having a partial sequence of IKKα and the like.

RNAi (RNA interference) is a phenomenon in which double-stranded RNA introduced into cells suppresses the expression of a gene having the same sequence as that of the RNA. Specific examples of a substance that inhibits IKKα expression via RNAi include siRNA and shRNA described below.

siRNA is an abbreviation of short interfering RNA and means double-stranded RNA with a length between approximately 21 and 23 bases. siRNA may be in any form, as long as it can induce RNAi. An example of such siRNA is siRNA obtained via chemical or biochemical synthesis or *in vivo* synthesis, or a short-chain double-stranded RNA or the like of 10 or more base pairs, which is produced by *in vivo* degradation of double-stranded RNA of approximately 40 or more bases. An siRNA sequence preferably matches 100% a partial sequence of IKKα mRNA. However, a 100% match is not always required.

A homologous region between the nucleotide sequence of siRNA and the nucleotide sequence of an IKKα gene preferably contains no translation initiation region of the IKKα gene. A sequence with homology is preferably located 20 bases and more preferably 70 bases away from the translation initiation region of the IKKα gene. A sequence with homology may be a sequence in the vicinity of the 3' terminus of the IKKα gene, for example.

As a substance that inhibits IKKα expression via RNAi, siRNA-generating dsRNA of approximately 40 or more bases may also be used. For example, double-stranded-portion-containing RNA or an altered product thereof can be used, which contains a sequence having approximately 70% or more, preferably 75% or more, more preferably 80% or more, more preferably 85% or more, further more preferably 90% or more, particularly preferably 95% or more, most preferably 100% homology with a portion of the nucleic acid sequence of the IKKα gene. A sequence portion having homology comprises generally at least 15 nucleotides or more, preferably approximately 19 nucleotides or more, more preferably at least 20 nucleotides or more, and further preferably 21 nucleotides or more.

As a substance that inhibits IKKα expression via RNAi, shRNA (short hairpin RNA) comprising a short hairpin structure having a projection at the 3' terminus can also be used. "shRNA" means a molecule of approximately 20 or more base pairs, which is formed when a single-stranded RNA contains partially palindromic nucleotide sequences so that it forms a double-stranded structure within the molecule leading to the formation of a hairpin-like structure. Furthermore, preferably, shRNA has a projecting 3' terminus. The length of a double-stranded portion is not particularly limited and is preferably 10 or more nucleotides and more preferably 20 or more nucleotides. Here, the projecting 3' terminus is preferably a DNA, more preferably a DNA of at least 2 or more nucleotides, and further preferably a DNA of 2 to 4 nucleotides.

A substance that inhibits IKKα expression via RNAi may be artificially and chemically synthesized. The substance can also be prepared through *in vitro* RNA synthesis using DNA with a hairpin structure wherein a sense strand DNA sequence and an antisense strand DNA sequence are linked in a reverse manner and T7 RNA polymerase. In the case of *in vitro* synthesis, antisense and sense RNAs can be synthesized using T7 RNA polymerase, a T7 promoter, and a template DNA. After *in vitro* annealing thereof, the resultant is introduced into cells. RNAi is thus induced and the expression of IKK α is suppressed. For example, such introduction into cells can be performed using a calcium phosphate method, a method using various transfection reagents (e.g., oligofectamine, lipofectamine, and lipofection), or the like.

As a substance that inhibits IKK α expression via RNAi, an expression vector containing a nucleic acid sequence encoding the above siRNA or shRNA may also be used. Furthermore, cells containing such expression vector may also be used. Types of the above expression vector or cells are not particularly limited. Expression vectors or cells that have already been used as medicaments are preferred.

In the present invention, IKK α or a substance that increases IKK α expression can be used as a agent for inducing interferon-α production. Examples of such substance that increases IKK α expression include proteins such as transcription factors and low-molecular-weight compounds.

The route of administration for the agent for controlling interferon-α of the present invention is not particularly limited and may be oral administration or parenteral administration (e.g., intravenous administration, intramuscular administration, subcutaneous administration, intradermal administration, transmucosal administration, intrarectal administration, intravaginal administration, local administration to affected area, and skin administration). The agent may be administered via any of these administration routes. Examples of dosage forms appropriate for oral administration include solid forms and liquid forms. Examples of dosage forms appropriate for parenteral administration include forms of injections, infusions, suppositories, external preparations, eye drops, nasal preparations, and the like. The agent for controlling interferon-α of the present invention may be in a dosage form of a sustained preparation. The agent for controlling interferon-α of the present invention may be supplemented with pharmaceutically acceptable additives depending on its dosage form, if necessary. Specific examples of pharmaceutically acceptable additives include an excipient, a binder, a disintegrator, a lubricant, an antioxidant, a preservative, a stabilizer, an isotonizing agent, a colorant, a taste corrigent, a diluent, an emulsifier, a suspending agent, a solvent, a filler, an augmentor, a buffer agent, a delivery vehicle, a diluent, a carrier, an excipient, and/or a pharmaceutical adjuvant.

The agent for controlling interferon-α of the present invention in a solid dosage form for oral administration can be prepared by, for example, adding an excipient to an agent for inhibiting IKKα, IKKα, or a substance that increases IKKα expression as an active ingredient and adding, if necessary, an additive for formulation such as a binder, a disintegrator, a lubricant, a colorant, or a taste corrigent, and then preparing by a conventional technique the agent in the form of tablets, fine granules, powdered drugs, or capsules. The agent for controlling interferon-α of the present invention in a liquid dosage form for oral administration can be prepared by a conventional technique as liquids for internal use, syrups, elixirs or the like through addition of one, two, or more types of additive for formulation, such as a taste corrigent, a stabilizer, or a preservative to an agent for inhibiting IKKα, IKKα or a substance that increases IKKα expression as an active ingredient.

A solvent to be used for prescribing the agent for controlling interferon-α of the present invention as a liquid formulation may be either aqueous or non-aqueous. A liquid formulation can be prepared by a method known in the art. For example, an injection can be prepared by dissolving in a physiological saline solution, a buffer such as PBS, or a solvent such as sterilized water, filter-sterilizing the resultant using a filter or the like, and then filling a sterile container (e.g., ampule) with the resultant. Such injection may further contain a conventionally used pharmaceutical carrier, if necessary. Furthermore, an administration method that uses noninvasive catheters may also be employed. Examples of a carrier that can be used in the present invention include a neutrally buffered physiological saline solution and a physiological saline solution containing serum albumin.

Types of means employed for gene delivery, such as siRNA of IKKα or an siRNA expression vector are not particularly limited, as long as RNA encoding siRNA of IKKα or an siRNA expression vector is expressed within cells used herein. For example, gene introduction methods using a viral vector and a liposome can be employed. Examples of viral vectors include vectors of animal viruses such as retrovirus, vaccinia virus, adenovirus, and Semliki Forest virus.

A substance that inhibits IKKα expression via RNAi may be directly injected into cells.

The dose of the agent for controlling interferon-α of the present invention can be determined by persons skilled in the art in view of purposes of use, disease severity, patient age, body weight, sex, and past medical history, or types of substance to be used as an active ingredient, for example. The dose of the agent for controlling interferon-α of the present invention ranges from approximately 0.1 ng to approximately 100 mg/kg/adult and preferably from approximately 1 ng to approximately 10 mg/kg/adult in terms of an amount of the active ingredient, for example. When the agent is administered in the form of a viral vector or a non-viral vector, the dose generally ranges from 0.0001 mg to 100 mg, preferably 0.001 mg to 10 mg, and more preferably 0.01 mg to 1 mg.

Administration frequency of the agent for controlling interferon-α of the present invention may range from once a day to once per several months, for example. When a substance that inhibits IKKα expression via RNAi is used, administration is preferably performed at a frequency of once a day to once every 3 days. This is because the effect of administration generally lasts for 1 to 3 days after administration. When an expression vector is used, preferable administration may also be performed approximately once a week.

The agent for controlling interferon-α of the present invention (an agent for suppressing interferon-α production or an agent for inducing interferon-α production) can be used for treating or preventing diseases associated with hyperproduction of interferon or diseases the treatment or the prevention of which can be expected by inducing interferon production *in vivo.*

Specific examples of such diseases include, various inflammatory conditions, collagen diseases, autoimmune diseases, various immune diseases, inflammation and pain particularly in the joint and muscle (e.g., chronic rheumatoid arthritis, rheumatoid spondylitis, osteoarthritis, and urarthritis), dermal inflammatory conditions (e.g., eczema), inflammatory conditions of eyes (e.g., conjunctivitis), lung disorders with inflammation (e.g., asthma and bronchitis), conditions of digestive organs with inflammation (e.g., aphthous ulcer, clone disease, atrophic gastritis, verrucous gastritis, ulcerative colitis, steatorrhea, ileitis terminalis, and irritable bowel syndrome), gingivitis (inflammation, pain, and swelling after operation or disorder), inflammation-related onset of fever, pain, other conditions, graft rejection, systemic erythematosus, scleroderma, polymyositis, polychondritis, periarteritis nodosa, necrotic vasculitis, reactive spondyloarthropathy, ankylosing spondylitis, chronic inflammatory conditions of kidney (e.g., glomerulonephritis, lupus erythematosus nephritis, and membranous nephritis), rheumatic fever, Sjogren's syndrome, Behcet's disease, thyroiditis, type I diabetes mellitus, dermatomyositis, chronic active hepatitis, myasthenia gravis, Graves' disease, multiple sclerosis, primary biliary cirrhosis, autoimmune blood disease (e.g., hemolytic anemia, true cytic anemia, idiopathic thrombocytopenia, and aplastic anemia), Hashimoto's disease, uveitis, contact dermatitis, psoriasis, Kawasaki disease, diseases associated with type I hypersensitivity (e.g., allergic asthma, atopic dermatitis, urticaria, allergic conjunctivitis, pollinosis, eczema, food hypersensitivity, and allergic rhinitis), shock (e.g., septic shock, anaphylactic shock, and adult respiratory distress syndrome), sarcoidosis, Wegener's granulomatosis, Hodgkin's disease, and cancers (e.g., lung cancer, gastric cancer, colon cancer, and hepatic cancer). Further examples of the same include various microbial infections, particularly infections due to various viruses, such as acute infections (e.g., influenza virus, herpes simplex virus, and vesicular stomatitis virus), chronic infections (e.g., hepatitis B virus and hepatitis C virus), various bacterial infections, various fungal infections, and various parasitic infections.

Hereafter, the present invention is described in greater detail with reference to the following examples, although the present invention is not limited to these examples.

### Examples

### Example 1: Need for IKKα in induction of IFN-α production from bone marrow-derived in vitro dendritic cells via TLR7 and TLR9 stimulation

IKKα+/- mice were crossed to obtain fetuses on embryonic days 13.5 to 15.5. Mice having the genetic trait of IKKα +/+ or -/- were selected by PCR from the fetuses and then fetal liver cells were collected. Moreover, fetal liver cells were intravenously injected into irradiated wild-type C57BL6-CD45.1 mice. 6 to 10 weeks later, the mice were used as IKKα+/+ or IKKα-/- bone marrow chimeric mice.

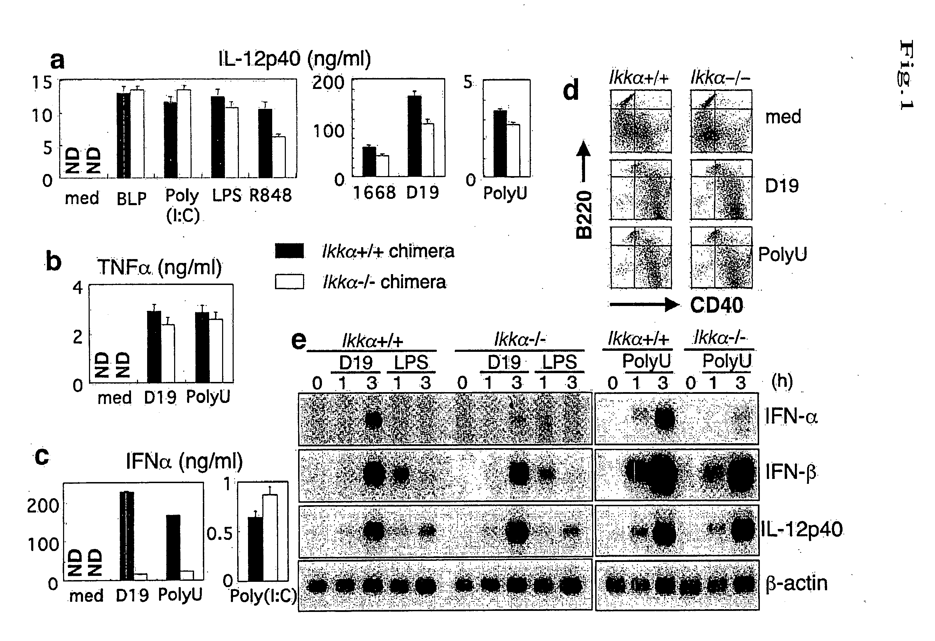

The chimeric mouse bone marrow cells were cultured at a concentration of 5x10⁶ cells/ml in RPMI1640 medium containing 10% FCS and 100 ng/ml human Flt3 ligand (Peprotech) for 6 to 8 days. Thus, Flt3L-induced bone marrow dendritic cells (Flt3L BM DC) were prepared. The Flt3L BM DC was not stimulated (med) or was stimulated for 20 to 24 hours with 100 ng/ml Bacterial lipopeptide (BLP, Invivogen), 50 µg/ml polyinosinic-polycytidylic acid (poly (I : C), Amersham) (however, as shown in Fig. 1c, poly (I : C) with a final concentration of 25 µg/ml prepared by mixing with lipofectamine 2000 (Invitrogen) was used), 100 ng/ml Salmonella Minnesota Re595-derived LPS (Sigma), 100 nM R848 (Invivogen), synthetic DNA (1 µM 1668: 5'-tccatgacgttcctgatgct-3' (SEQ ID NO: 1), 3 µM D19: 5'-ggTGCATCGATGCAgggggG-3' (SEQ ID NO: 2) (in which small letters denote phosphorothioate DNA and capital letters denote phosphodiester DNA), and polyuridylic acid (PolyU) with a final concentration of 20 µg/ml which was prepared by mixing it with Lipofectamine 2000 (Invitrogen). The amounts of IL-12p40 and TNF-α in the culture supernatants were measured by ELISA (Genzyme Techne). The amount of IFN-α was measured by ELISA (PBL) (Fig. 1a, b, and c).

Wild-type Flt3L-induced bone marrow dendritic cells (Flt3L BM DC) produced IL-12p40 in response to bacterial lipopeptide (BLP, TLR2 ligand), poly (I : C) (TLR3 ligand), LPS (TLR4 ligand), R848 (TLR7 ligand), and ODN1668 (TLR9 ligand). IKKα -/- Flt3L BM DC also produced IL-12p40 in response to these TLR agonists. The amount of IL-12p40 induced via TLR7 or TLR9 signaling decreased slightly, but significantly increased after stimulation (Fig. 1a). No significant differences were confirmed between the wild type and IKKα -/- Flt3L BM DC in terms of the amount of TNF-α induced via TLR7 or TLR9 signaling (Fig. 1b). Furthermore, D/A-type CpG DNA (D19) could induce IFN-α production in wild-type Flt3L BM DC, but the induction was significantly damaged in IKKα-/- Flt3L BM DC (Fig. 1c). Poly U-induced IFN-α production was also significantly damaged in IKKα -/- Flt3L BM DC (Fig. 1c). Meanwhile, IFN-α production via stimulation with poly (I : C) was normal (Fig. 1c).

Furthermore, CD40 expression and B220 expression were analyzed by FACS (Fig. 1d). The proportions of two types of dendritic cells, B220⁺ (PDC) and B220⁻ (general DC), were normal. No significant differences were confirmed between IKKα +/+ and IKKα -/- Flt3L BM DC in terms of induction of CD40 expression in each DC type by TLR7 and TLR9 (Fig. 1d).

To analyze the expression of the IFN-α, IFN-β, or IL-12p40 gene in Flt3L BM DC, Flt3L BM DC was stimulated with LPS, D19, or PolyU, RNA was prepared, and then Northern blot analysis was performed using the IFN-α, IFN-β, and IL-12p40 genes as probes (Fig. 1e). IFN-α gene induction via TLR7 or TLR9 signaling was significantly deteriorated in IKKα-/- Flt3L BM DC. However, increases in IFN-β and IL-12p40 gene expression induced by TLR7 or TLR9 were equivalent among the wild type and IKKα -/- Flt3L BM DC (Fig. 1e). These results demonstrate that IKKα, is essential for TLR7- or TLR9-mediated IFN-α induction in *in vitro* dendritic cells.

### Example 2: Need for IKKα in induction of IFN-α production from in vivo dendritic cells via TLR7 or TLR9 stimulation

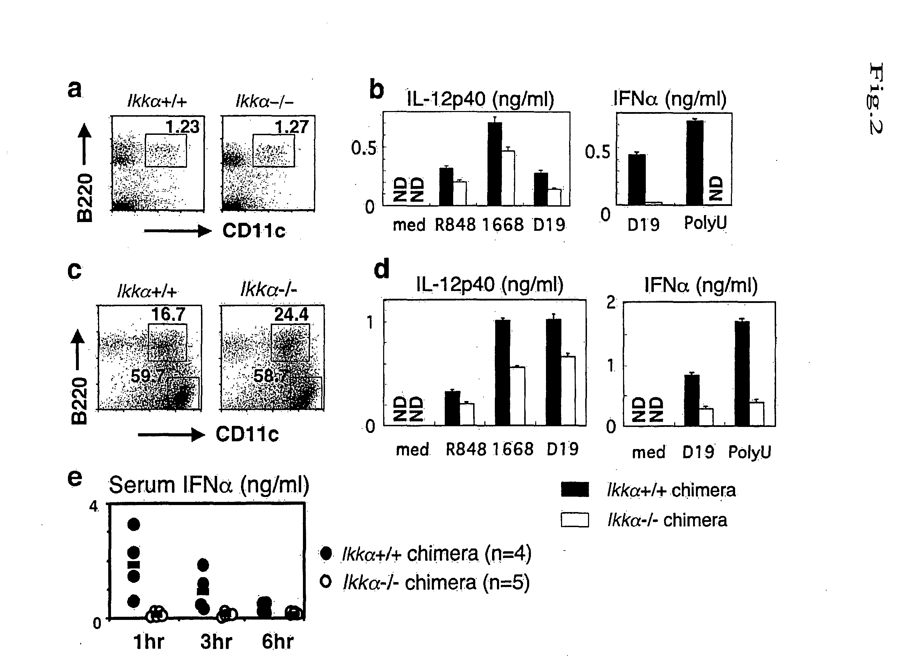

CD11c expression and B220 expression were analyzed by FACS in chimeric mouse bone marrow cells (Fig. 2a). As a result, both IKKα+/+ and IKKα-/- chimeras were found to contain CD11c⁺B220⁺ cells in bone marrow cells.

Bone marrow cells (1x10⁶ cells/ml) in RPMI1640 medium containing 10% FCS were not stimulated or were stimulated for 20 to 24 hours with polyU with a final concentration of 25 µg/ml prepared by mixing 100 nM R848, 1 µM 1668, 3 µM D 19, and Lipofectamine 2000 (Invitrogen). After stimulation, the amounts of IL-12p40 in the culture supernatants were measured by ELISA (Genzyme Techne) and the amounts of IFN-α were measured by ELISA (PBL) (Fig. 2b). TLR7- or TLR9-induced IFN-α production significantly decreased in IKKα -/- chimeric bone marrow cells. In contrast, IL-12p40 induction decreased slightly (Fig. 2b).

CD11c positive cells were prepared from chimeric mouse spleen cells by magnetic cell sorting (MACS) using magnetic beads. CD11c expression and B220 expression in the cells were analyzed by FACS (Fig. 2c). The numbers of B220 positive cells among CD11c positive cells were equivalent among IKKα +/+ and IKKα -/- chimeric spleen cells (Fig. 2c).

Furthermore, the CD11c positive spleen cells were stimulated under conditions similar to those for bone marrow cells. The production amounts of IL-12p40 and IFN-α were measured by ELISA (Fig. 2d). The production amount of IL-12p40 in IKKα -/-CD11c positive spleen cells was decreased slightly more than that in IKKα +/+ CD11c positive spleen cells. The amount of IFN-α production induced by TLR7/9 in IKKα -/-CD11c positive spleen cells was significantly decreased (Fig. 2d).

50 nmol of R848 was intravenously injected per chimeric mouse. Blood was drawn at 1, 3, and 6 hours after intravenous injection. IFN-α concentration in serum was measured by ELISA (Fig. 2e). Elevation of IFN-α concentration in serum, which was induced by TLR-7 ligand (R848), depended on production from PDC but the concentration was significantly decreased in IKKα -/- chimeras (Fig. 2e).

### Example 3: IKKα functions in activation of IFN-α promoter via MyD88-dependent pathway

### (1) Preparation of plasmid

pUNO-MyD88 and pUNO-TRAF6: They were purchased from Invivogen.
pUNO: pUNO-TRAF6 was cleaved with *Age* I + *Nhe* I, inserts were removed, self annealing was performed, and then the resultant was used as a control vector.
pEF-BOS-FLAG-mIRF-7: PCR was performed using primers 5'-GGGTCGACATGGACTACAAAGACGATGACGACAAGGCTGAAGTGAGGGGG GTCCAGCGA-3' (SEQ ID NO: 3) and 5'-GGGCGGCCGCTCAAGGCCACTGACCCAGGTCCATGAG-3'(SEQ ID NO: 4), so that IRF7 cDNA was amplified from the cDNA library of GM-CSF-induced dendritic cells derived from mouse bone marrow. The product was inserted into the *Sal* I*-Not* I site of plasmid pEF-BOS (S. Mizushima, S. Nagata. Nucleic Acids Res. 18: 5322, 1990). pSRα-6myc-mIKKα: pSRα-6myc-mIKKα was provided by Prof. Mitsuru Matsumoto, The University of Tokushima, and used as an expression vector for wild-type IKKα (IKKα-WT).
pSRα-6myc-mIKKα(K44A): Based on pSRα-6myc-mIKKα, *in vitro* mutagenesis was performed so as to convert lysine residue 44 into alanine residue. The resultant was used as an expression vector for IKKα (IKKα-KD) lacking kinase activity.
pSRα-6myc: mIKKα was cleaved from pSRα-6myc-mIKKα using *EcoR* I + *Xho* I, the terminus was repaired, and then self annealing was performed.
pIFN-α4-luc: An IFN-α4 promoter region was amplified from C57BL6 mouse DNA by PCR using a sense primer 5'-CCCCCACACTTTACTTTTTTGACAGAA-3' (SEQ ID NO: 5) and an antisense primer 5'-TACAGGTTCTCTGAGAGCCTGCTGTGT-3' (SEQ ID NO: 6). The promoter region was subcloned into the *Xho* I*-Hind* III site of pGL3 (Promega), so that pIFN-α4-luc was prepared.
pELAM1-luc: pELAM1-luc was provided by Dr. D. T. Golenbock. R. L. Delude et al. J. Immunol. 161: 3001, 1998.
pIFN-β-luc: According to Sato et al. Immunity 13: 539, 2000, an IFN-β promoter region was amplified by PCR and then the product was subcloned into the *Xho* I-*Hind* III site of pGL3 (Promega), so that pIFN-β-luc was prepared.
pRL-TK: pRL-TK was purchased from Promega.

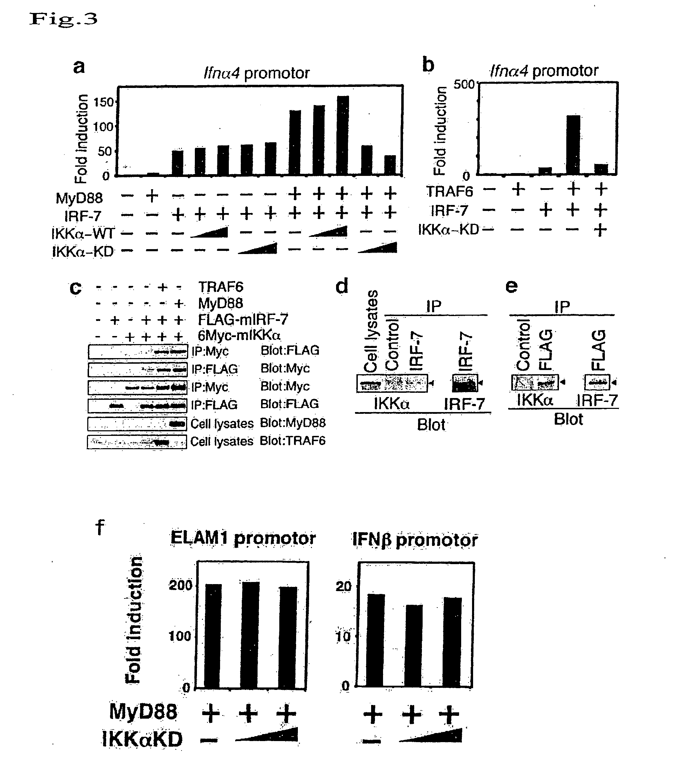

### (2) Luciferase assay (Figs. 3a, b, and f)

The cells of cell line 293T derived from human kidney were cultured at a concentration of 2x10⁵ cells/ml in DMEM medium containing 10% FCS on a 24-well plate at 0.35 ml/well. 24 hours later, various combinations of pUNO, pUNO-MyD88 (Invivogen), pUNO-TRAF6, pEF-BOS-Flag-mIRF-7, pSRα-6myc, pSRα-6myc-mIKKα, and pSRα-6myc-mIKKα (K44A) were added with reporter gene pIFN-α4-luc (Figs. 3a and b), pELAM1-luc (Fig. 3f), or pIFN-β-luc (Fig. 3f), and pRL-TK (Promega). Gene introduction was performed using lipofectamine 2000 (Invivogen). 18 to 24 hours later, cell extracts were prepared and then luciferase activity was determined using a Dual-Luciferase Reporter Assay System (Promega) and 20/20ⁿ Luminometer (Turner Biosystem). To correct gene introduction efficiency, the thus obtained figures for firefly luciferase activity was divided by the figures for Renilla luciferase activity to obtain relative activity level. Finally, experimental data are represented via fold induction using a relative activity level (obtained when an empty vector alone had been introduced) designated as 1.

The expression of TLR adaptor MyD88 alone was unable to activate IFN-α promoter, but it was able to synergistically increase the IRF-7-induced activation of the promoter (Fig. 3a). IKKα-KD expression did not affect the activation by IRF-7 alone (Fig. 3a), but it successfully inhibited the synergistic effect due to MyD88, which was confirmed under co-existence with IRF-7 (Fig. 3a). Meanwhile, IKKα-WT expression slightly enhanced the synergistic effects of MyD88 and IRF-7 (Fig. 3a). Similar to MyD88, TRAF6, which is another adaptor molecule, could also activate IFN-α promoter synergistically with IRF-7 (Fig. 3b). IKKα-KD could also inhibit the synergistic effects exerted by such TRAF6 induction. MyD88 could also activate an NF-κB or IFN-β promoter, but the activity was not inhibited by IKKα-KD expression (Fig. 3f). Accordingly, it was demonstrated that through its kinase activity, IKKα is involved in IRF7's ability to activate an IFN-α promoter synergistically with MyD88 or TRAF6.

### (3) Association of IKKα with IRF7 (Figs. 3c, d, and e)

### (3-1) Association of IKKα with IRF7 in 293T cells (Fig. 3c)

2x10⁶ 293T cells were cultured overnight per 60-mm dish in DMEM medium containing 10% FCS. Gene introduction was performed using 5 µg of each plasmid identified below in total and lipofectamine 2000 (Invivogen). 20 hours after gene introduction, cells were collected, followed by cell extraction using a lysis solution (1% Nonidet P-40, 150 mM NaCl, 5 mM EDTA, 1 mM PMSF, 20 µg/ml aprotinin, 10 mM NaF, 10 mM β-glycerophosphate, 1 mM Na₃VO₄, and 20 mM Tris-HCl, pH 8.0). The resultant was mixed with protein G-Sepharose4FF beads for 1 hour. The supernatant was further mixed with protein G-Sepharose4FF beads bound with 6 µg/ml anti-Flag M2 mAb (Sigma-Aldrich) or 5 µg/ml anti-Myc-tag clone PL14 mAb (MBL, Japan) at 4°C for 12 hours. After the beads had been washed three times, elution was performed at 100°C for 5 minutes using Laemmli solution. The resultant was subjected to 10% SDS polyacrylamide gel electrophoresis and then transferred onto a PVDF membrane. Next, the membrane was mixed with biotin-labeled anti-Flag M2 mAb (Sigma-Aldrich) or biotin-labeled anti-Myc-tag clone PL14 mAb (MBL, Japan) at room temperature for 1 hour. After washing with TBS-T buffer (0.1% Tween20, 137 mM NaCl, 2.6 mM KCl, and 25 mM Tris-HCl, pH 7.0), the resultant was further mixed with streptavidin-HRP conjugate (Amersham bioscience) at room temperature for 30 minutes. After further washing, band detection was performed using an ECL system (PerkinElmer Life sciences).

As a result, it was confirmed that IKKα had been immunoprecipitated together with IRF-7 (Fig. 3c). Coexpression with MyD88 or TRAF6 can significantly enhance interaction between IRF7 and IKKα (Fig. 3c).

### (3-2) Association of IKKα with IRF-7 in Flt3L-induced BM DC (Fig. 3d)

Preparation of cell extracts, immunoprecipitation, and Western blot were performed in a manner similar to that in Fig. 3c using prepared wild-type Flt3L BM DC, rabbit anti-IRF-7 antibody (United States Biological), normal rabbit antibody IgG (Santa Cruz Biotechnology) as a control, and anti-IKKα antibody (M-280, Santa Cruz Biotechnology). HRP-labeled burro anti-rabbit IgG antibody (Amersham Bioscience) was used for detection of the band corresponding to IRF-7.

As a result, association of IKKα with IRF-7 was detected (Fig. 3d).

### (3-3) Association of IKKα with IRF7 in GM-CSF-induced BMDC (Fig. 3e)

GM-CSF-induced bone marrow dendritic cells (GM-CSF BM DC) were prepared by culturing bone marrow cells of IKKα+/+ or IKKα-/- chimeric mice at a concentration of 1x10⁶ cells/ml in RPMI1640 medium containing 10% FCS and 10 ng/ml GM-CSF (R&D) for 6 to 8 days. Furthermore, GM-CSF BM DC was forced to express IRF7 using a lentivirus vector pCSII-EF-FLAG-mIRF7-IRES2-Venus. pCSII-EF-FLAG-mIRF7-IRES2-Venus was constructed by inserting IRF7 cDNA prepared in Example 3(1) into a lentivirus vector pCSII-EF-MCS-IRES2-Venus (provided by Dr. Hiroyuki Miyoshi and Dr. Atsufumi Miyawaki, RIKEN). The GM-CSF-DC was subjected to preparation of cell extracts, immunoprecipitation, and Western blot in a manner similar to that of Fig. 3c using Anti-FLAG M2 mAb, anti-myc-tag clone PL14 mAb as a control thereto, an anti-IRF7 antibody, and an anti-IKKα antibody (M-280, Santa Cruz Biotechnology).

As a result, association of IRF7 with IKKα was detected (Fig. 3e).

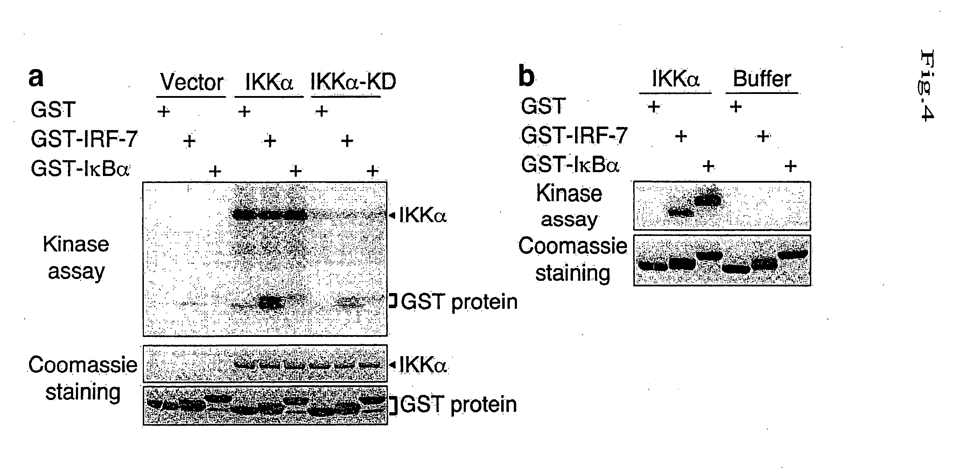

### Example 4: Phosphorylation of GST-IRF7 by IKKα (Fig. 4)

### (1) Preparation of plasmid

pGST-IRF7: A region corresponding to amino acids 422-457 of pEF-BOS-FLAG-mIRF-7 was amplified by PCR and then the product was subcloned into pGEX-5X-1 (Amersham Biosciences).
pGST-IκBα: A region corresponding to amino acids 5-55 of mouse IκBα was amplified by PCR and then the product was subcloned into pGEX-5X-1 (Amersham Biosciences).

### (2) Phosphorylation assay (Fig. 4)

pGEX-5X-1, pGST-IRF7, and pGST-IκBα were introduced into *Escherichia coli* BL21. GST protein (control), GST fusion IRF7, and GST fusion pGST-IκBα were purified using glutathione sepharose 4B affinity chromatography (Amersham Biosciences), and the resultants were then used as substrates. pSRα-6myc (control vector), pSRα-6myc-mIKKα, and pSRα-6myc-mIKKα (K44A) were introduced into 293T cells. Cell extracts were prepared and then subjected to immunoprecipitation using PL14. The resulting immunoprecipitates were each mixed with 2 µg of the substrate, so that reaction was performed at 37°C for 30 minutes in the presence of 20 mM HEPES-NaOH (pH 7.6), 10 mM MgCl₂, 2 mM MnCl₂, 50 mM NaCl, 0.1 mM Na₃VO₄, 10 mM β-glycerophosphate, 10 mM PNPP, 1 mM DTT, 10 mM NaF, 50 µM ATP, and 370 kBq [γ-³²P]ATP (Fig. 4a). After reaction, the resultants were subjected to 10% SDS polyacrylamide gel electrophoresis and then autoradiography. In addition, similar reaction was performed using 100 ng (60 units/mg protein) of recombinant human IKKα (Lake Placid) (Fig. 4b).

As a result, it was demonstrated that IRF7 was phosphorylated by IKKα (Fig. 4a).

### Industrial Applicability

The present invention makes it possible to control interferon-α production. The agent for controlling interferon-α of the present invention can be used for treating or preventing various diseases, since interferon-α production suppressed or induced by the use of the agent is effective for treating or preventing such diseases.

### Brief Description of the Drawings

Fig. 1 shows experimental results of examining the need for IKKα in induction of IFN-α production from bone-marrow-derived *in vitro* dendritic cells by TLR7 or TLR9 simulation.
Fig. 2 shows experimental results of examining the need for IKKα in induction of IFN-α production from *in vivo* dendritic cells by TLR7 or 9 stimulation.
Fig. 3 shows experimental results of examining IKKα functions in IFN-α promoter activation via MyD88-dependent pathway and association of IKKα with IRF7.
Fig. 4 shows experimental results showing GST-IRF7 phosphorylation by IKKα.

## Claims

1. An agent for suppressing interferon-α production which comprises an agent for inhibiting IKKα.

2. The agent for suppressing interferon-α production of claim 1 wherein the agent for inhibiting IKKα is a substance that inhibits IKKα expression or an IKKα mutant lacking kinase activity.

3. A method for suppressing interferon-α production from cells, which comprises inhibiting intracellular IKKα activity.

4. An agent for inducing interferon-α production which comprises IKKα or a substance that increases IKKα expression.

5. A method for inducing interferon-α production from cells, which comprises administering IKKα or a substance that increases IKKα expression to cells.


SEQUENCE LISTING

<110> RIKEN

<120> Interferon-alpha Regulator

<130> P22127EP

<140> EP 06747113.6
<141> 2006-06-02

<150> JP 163647/2005
<151> 2005-06-03

<160> 6

<170> PatentIn version 3.3

<210> 1
<211> 20
<212> DNA
<213> Artificial

<220>
<223> Description of Artificial Sequence: Synthetic DNA

<400> 1
tccatgacgt tcctgatgct
20

<210> 2
<211> 20
<212> DNA
<213> Artificial

<220>
<223> Description of Artificial Sequence: Synthetic DNA

<400> 2
ggtgcatcga tgcagggggg
20

<210> 3
<211> 59
<212> DNA
<213> Artificial

<220>
<223> Description of Artificial Sequence: Synthetic DNA

<400>  3
gggtcgacat ggactacaaa gacgatgacg acaaggctga agtgaggggg gtccagcga
     59


<210>  4
<211>  37
<212>  DNA
<213>  Artificial

<220>
<223>  Description of Artificial Sequence: Synthetic DNA

<400>  4
gggcggccgc tcaaggccac tgacccaggt ccatgag
     37


<210>  5
<211>  27
<212>  DNA
<213>  Artificial

<220>
<223>  Description of Artificial Sequence: Synthetic DNA

<400>  5
cccccacact ttactttttt gacagaa
     27


<210>  6
<211>  27
<212>  DNA
<213>  Artificial

<220>
<223>  Description of Artificial Sequence: Synthetic DNA

<400>  6
tacaggttct ctgagagcct gctgtgt
     27